# FASCICULE DE BREVET EUROPEEN

(11) **EP 2 480 609 B1**
(45) Date de publication et mention de la délivrance du brevet: **27.12.2017**
(21) Numéro de dépôt: 10757211.7
(22) Date de dépôt: 24.09.2010
(51) Int. Cl.: C09B 57/02, C09B 69/10

(54) **POLYMERES FLUORESCENTS DE COMPOSES DE LA 7-HYDROXYCOUMARINE, CAPTEURS CHIMIQUES LES COMPRENANT, ET COMPOSE FLUORESCENT POLYMERISABLE DE LA 7-HYDROXYCOUMARINE**
FLUORESZENZPOLYMERE VON 7-HYDROXYCUMARIN-VERBINDUNGEN, CHEMISCHE SENSOREN DAMIT UND POLYMERSIERBARE FLUORESZENTE 7-HYDROXYCUMARIN-VERBINDUNG
FLUORESCENT POLYMERS OF 7-HYDROXYCOUMARIN COMPOUNDS, CHEMICAL SENSORS INCLUDING SAME, AND POLYMERISABLE FLUORESCENT 7-HYDROXYCOUMARIN COMPOUND

(30) Priorité: 25.09.2009 FR 0956656
(43) Date de publication de la demande: 01.08.2012
(73) Titulaire: Commissariat à l'Énergie Atomique et aux Énergies Alternatives, 75015 Paris (FR)
(72) Inventeur: BUVAT, Pierrick, 37250 Montbazon (FR); MALOSSE, Lucie, 63100 Clermont-ferrand (FR); SIOVE, Alain, 95230 Soisy-sous-montmorency (FR); ADES, Dominique, 95240 Cormeilles-en-parisis (FR)
(74) Mandataire: Ilgart, Jean-Christophe
(86) Numéro de dépôt international: PCT/EP2010/064181
(87) Numéro de publication internationale: WO 2011/036269

(56) Documents cités:
- US-A1- 2007 254 859
- KREJCOVES J ET AL: "The Preparation and Characterization of Some Novel Fluorescence Labels Derived from 7-Substituted 2H-1-Benzopyrane-2-Ones", COLLECTION OF CZECHOSLOVAK CHEMICAL COMMUNICATIONS, INSTITUTE OF ORGANIC CHEMISTRY & BIOCHEMISTRY, PRAGUE; CZ, vol. 44, 1 janvier 1979 (1979-01-01), pages 2211-2220, XP002108459, ISSN: 0010-0765

## Description

### DOMAINE TECHNIQUE

L'invention concerne des polymères fluorescents susceptibles d'être obtenus par polymérisation de composés fluorescents, polymérisables de la 7-hydroxycoumarine, et plus particulièrement de l'umbelliférone-4-acétate d'éthyl-2-méthacrylate.

Plus précisément, l'invention concerne des polymères de dérivés fluorescents, notamment polaires et hydrophobes de la 7-hydroxycoumarine comprenant en position 4, un substituant polymérisable qui ne modifie pas ou qui ne modifie pas de manière substantielle les propriétés électroniques et la photoluminescence du noyau coumarine.

L'invention a également trait à un nouveau composé fluorescent polymérisable de la 7-hydroxycoumarine et à sa préparation.

L'invention a, en outre, trait à des capteurs comprenant ces polymères fluorescents en tant que matériau sensible, notamment pour la détection et/ou le dosage de composés nitrés ou organophosphorés, d'explosifs et de composés toxiques.

L'invention a enfin trait à l'utilisation de ces polymères fluorescents pour la réalisation de sondes fluorescentes, de marqueurs biologiques, et de matériaux fluorescents, photoluminescents et/ou radioluminescents et/ou cathodoluminescents.

Le domaine technique de l'invention peut ainsi, de manière générale, être défini comme celui des polymères fluorescents.

### ÉTAT DE LA TECHNIQUE ANTÉRIEURE

Aujourd'hui, on assiste à une demande croissante en polymères fluorescents dans des secteurs très varies. Ainsi, dans le domaine de la sécurité, le marquage fluorescent présente un intérêt pour lutter contre la contrefaçon [1]. Dans le domaine de la criminalistique, il est mis en oeuvre dans les enquêtes policières [2]. Les polymères fluorescents sont aussi utilisés dans les capteurs chimiques pour la détection de polluants ou de composés toxiques dans l'eau ou dans l'air [3] [4] [5] et [6].

Dans le domaine de l'imagerie, notamment par microscopie confocale [7] ou microscopie de fluorescence, les polymères fluorescents peuvent également trouver des applications.

Dans les domaines thérapeutiques ou pharmaceutiques, les polymères fluorescents peuvent être couplés à un ligand biologique et utilisés ainsi dans des tests de détection de molécules cibles [8].

Dans le domaine des arts graphiques, ils peuvent être utilisés dans des peintures, des encres d'impression sur textile ou pour gravure [9].

On recense également dans la littérature d'autres applications originales des polymères fluorescents telles que celle décrite par Uchiyama et al. [10] qui ont mis au point des thermomètres moléculaires fluorescents en couplant le poly(N-isopropylacrylamide), connu pour subir une transition de phase en solution aqueuse vers 32°C, avec un dérivé fluorescent de type benzofurazane, dont la fluorescence depend de la polarité du solvant.

Si l'on s'intéresse maintenant aux coumarines, l'umbelliférone (ou 7-hydroxycoumarine) présente une émission de fluorescence bleue (300, 305 et 325 nm) lorsqu'elle est excitée par une radiation située dans l'ultraviolet. Nombre de ses dérivés ont une grande importance en physique, en chimie, en biologie et en médecine et ont été utilisés pour diverses applications [11].

En particulier, les propriétés photochimiques et photophysiques dans l'état excité de la 4-méthylumbelliférone (4MU) sont connues depuis longtemps et ce fluorophore a été utilisé comme sonde de pH et comme colorant laser [12] [13] et [14].

Dans le domaine biomédical, la 4MU s'est révélée également être un inhibiteur de la synthèse de l'acide hyaluronique [16] [17] [18], qui est l'un des principaux composants de la matrice extracellulaire qui contribue de façon significative à la prolifération et à la migration des cellules et qui se trouve ainsi impliqué dans la progression de certaines tumeurs malignes. Une récente revue de Yu et al. [19] décrit par ailleurs l'étude de plus de 150 dérivés de type coumarine et leur efficacité en tant qu'agents anti-VIH. La 4- heptadécyl-7-hydroxycoumarine, lipophile, a quant à elle été utilisée comme sonde pour étudier les propriétés de bicouches phospholipidiques à l'interface eau/lipide ou pour mesurer des différences de pH aux interfaces membranaires [20].

Faciles à coupler à d'autres entités par une chimie conventionnelle, les coumarines sont attractives de par leurs propriétés originales, à savoir notamment l'absorption et l'émission de lumière modulables, et la photodimérisation réversible. L'idée de les utiliser dans les polymères, pour synthétiser des architectures macromoléculaires à propriétés spécifiques, est donc apparue rapidement [11].

Par exemple, les propriétés de photodimérisation et photoclivage des coumarines ont été exploitées pour fabriquer des polymères à cristaux liquides et des polypeptides biodégradables. D'autres utilisations ont eu pour but d'obtenir des polymères « électroluminescents, ou encore de collecter et transférer l'énergie solaire.

Pour obtenir des polymères fluorescents, il existe plusieurs procédés. Le plus simple consiste à disperser des molécules fluorescentes dans une matrice de type polymère. Campbell et Barlett [7] ont ainsi synthétisé des sphères de poly(méthylméthacrylate) (PMMA) monodispersées par copolymérisation de méthacrylate de méthyle et d'acide méthacrylique en présence d'un colorant non polymérisable. Des colloïdes de PMMA fortement fluorescents ont ainsi été obtenus.

Molecular Probes® [21] commercialise des microsphères de polystyrène de tailles variées et de couleurs différentes suivant la nature du colorant qui y est dispersé (FluoSpheres®). Ces billes fluorescentes ont été utilisées comme traceurs de cellules microinjectables, comme marqueurs d'antigènes, pour la cytométrie de flux, ou encore pour étudier les phénomènes de phagocytose ou mesurer le débit sanguin dans les tissus. Bien que ces microsphères présentent une grande brillance et ne semblent pas subir de photoblanchiment notable, le fait que le colorant ne soit pas fixé de façon covalente à la matrice implique un hétérophasage et un risque de relargage du colorant dans le milieu biologique. Le greffage covalent du fluorophore est donc préférable pour éviter ces inconvénients.

Un autre procédé recensé consiste donc à « post-fonctionnaliser » des chaînes polymères non fluorescentes à partir de substituants permettant un couplage réactif avec un fluorophore. Saegusa [22] a ainsi décrit la synthèse de poly-oxazolines par polymérisation du 2-méthyl-2- oxazoline par ouverture de cycle, puis la fonctionnalisation du polymère par hydrolyse du groupe méthyle latéral, suivie d'un couplage avec l'acide 7-coumaryloxyacétique.

De même, le document de Rhum et Matthews [23] a trait à la copolymérisation de l'hydroxyéthylméthacrylate (HEMA) et du méthylméthacrylate (MMA), suivie du couplage du groupe hydroxyle de HEMA avec la 4- carboxyméthyl-umbelliférone, donnant ainsi un indicateur de pH non hydrosoluble.

Autrement dit, le document [23] (US-A-5,019,350) décrit des polymères fluorescents qui sont utiles comme indicateurs pour la détermination de la concentration des substances dissoutes dans un milieu aqueux.

En particulier, ce document décrit la synthèse d'un polymère fluorescent à partir de l'acide umbelliférone-4-acétique (CMU) et du polymère poly(HEMA-MMA) où HEMA est le méthacrylate d'hydroxyéthyle et MMA est le méthacrylate de méthyle.

De façon similaire, le document de Bouma et Celebuski [24] concerne des dérivés de la 7-hydroxycoumarine ayant des substituants en position 4.

Ces substituants comportent des groupements fonctionnels permettant de les coupler à des molécules biologiques, il s'agit typiquement de substituants à terminaison -OH, -SH ou -NH₂.

Cependant, ce procédé de « post-fonctionnalisation » ne s'applique qu'à un nombre restreint de polymères ayant les groupements fonctionnels adéquats tels que -COOH, -OH, -SH, -NH₂, ou -NCO et ne permet pas le contrôle, ni du taux ni de la répartition, du greffage du fluorophore sur le polymère.

Il est à noter que ces groupements fonctionnels ne sont pas des groupements que l'on peut qualifier de groupements polymérisables.

Les fluorophores pouvant être utilisés directement comme monomère ou comonomère, présentent l'avantage de résoudre les inconvénients évoqués ci-dessus. Pitschke et al. [25] décrivent la synthèse d'un monomère dérivé de la 7-aminocoumarine, substitué en position 3 par un groupement polymérisable styrénique (Formule la ci-dessous). Toutefois, la substitution du noyau coumarine en position 3 est connue pour modifier profondément les propriétés électroniques du noyau coumarine d'origine [23].

Rathbone *et al.* [26] décrivent quant à eux, l'utilisation du monomère 7-hydroxy- 4-méthylcoumarine acrylate (Formule 1b ci-dessous) pour la synthèse de polymères à empreinte moléculaire fluorescents. Cependant le blocage de la fonction phénol en position 7 par le groupement acrylate fait perdre un site d'interaction par liaison hydrogène essentiel pour de nombreuses applications telles que notamment les mesures de pH. 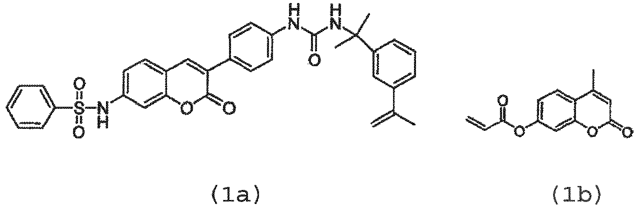 Structure des monomères fluorescents décrits par Pitschke *et al*. (1a) et par Rathbone *et al.* (1b).

Le document de KREJCOVES J. et al. : « The preparation and characterization of some novel fluorescence labels derived from 7-substituted 2H-1-Benzopyrane-2-Ones", Collection of Czechoslovak Chemical Communications, Institute of Organic Chemistry & Biochemistry, Prague; CZ, vol.44, 1er Janvier 1979, pages 2211-2220 divulgue la préparation de composés fluorescents dérivés des coumarines. En particulier, le composé XVIII de ce document est un dérivé de la 7-OH coumarine.

Le document US-A1-2007/254859 divulgue notamment un dérivé de la 7-OH coumarine couplé avec le polyéthylèneglycol (PEG1000).

En résumé, la dispersion de colorant dans les polymères n'est pas satisfaisante car dans ce cas le colorant n'est pas greffé par covalescence au polymère, ce qui ne permet pas d'assurer une durabilité suffisante et entraîne un risque de relargage du colorant.

Par ailleurs, le couplage réactif d'un colorant sur des chaînes polymères préformées d'une part, ne permet pas de contrôler le greffage (taux et répartition) et donc les propriétés physicochimiques des matériaux et d'autre part, restreint à la fois le nombre de polymères et le type de colorant utilisables aux composés comportant des fonctions réactives tels que -OH, -NH₂, -SH, -NCO, -COOH.

Enfin, nous avons constaté que seul un nombre restreint de fluorophores directement polymérisables est commercialisé à ce jour et que les rares dérivés de la coumarine modifiés chimiquement pour devenir polymérisables ne présentent plus les propriétés originelles de la coumarine.

Il existe donc au regard de ce qui précède un besoin pour un composé fluorescent ou fluorophore dérivé de la coumarine et notamment de la 7-hydroxycoumarine qui soit directement polymérisable c'est-à-dire pourvu d'un substituant polymérisable, mais dans lequel les propriétés électroniques et la photoluminescence du noyau coumarine ne sont pas ou substantiellement pas affectées par ce substituant polymérisable.

En d'autres termes, il existe un besoin pour un composé fluorescent polymérisable dérivé de la coumarine en tant que fluorophore de base dans lequel les propriétés originelles, notamment les propriétés électroniques et de photoluminescence de ce fluorophore de base sont intégralement ou du moins en grande partie conservées.

En particulier, il serait intéressant de pouvoir disposer d'un composé, monomère polymérisable dérivé de la 7-hydroxycoumarine dans lequel toutes les propriétés de la 7-hydroxycoumarine seraient conservées et dans lequel la fonction phénol en position 7 du cycle coumarine, dont on connaît l'importance dans de nombreuses applications, serait laissée intacte.

En outre, il existe un besoin pour un tel composé fluorescent polymérisable qui puisse être facilement polymérisé, notamment par les procédés de polymérisation radicalaire contrôlée ou non et qui puisse donc se lier de manière covalente à des chaînes d'homopolymères et/ou de copolymères par exemple vinyliques, acryliques ou styréniques.

Le but de l'invention est de fournir un composé polymérisable encore appelé monomère polymérisable qui réponde entre autres aux besoins énumérés plus haut.

Le but de l'invention est encore, de fournir un composé polymérisable qui ne présente pas les inconvénients, défauts, limitations et désavantages des composés polymérisables mentionnés plus haut et qui résolve les problèmes de ces composés.

### EXPOSÉ DE L'INVENTION

Ce but, et d'autres encore, sont atteints, conformément à l'invention par un composé fluorescent polymérisable, de la 7-hydroxycoumarine qui est l'umbelliférone-4-acétate d'éthyl-2-méthacrylate.

Le composé selon l'invention n'a pas été décrit dans l'art antérieur.

Le composé selon l'invention peut être défini comme un dérivé fluorescent polymérisable, polaire, de la 7-hydroxycoumarine qui comporte en position 4, un substituant polymérisable qui ne modifie pas, ou qui ne modifie pas de manière substantielle les propriétés électroniques et la photoluminescence du noyau coumarine de base.

Le composé selon l'invention peut être considéré en particulier comme un dérivé de la 4-méthylumbelliférone encore appelée 7-hydroxy-4-méthylcoumarine.

Le composé selon l'invention ne présente pas les inconvénients des fluorophores à base de coumarine connus, décrits plus haut et apporte une solution aux problèmes posés par ces fluorophores à base de coumarine connus.

Ainsi, dans le composé selon l'invention d'une part les propriétés électroniques et de photoluminescence du fluorophore d'origine, à savoir la 7-hydroxycoumarine, sont conservées grâce à la fonctionnalisation du noyau coumarine spécifiquement en position 4, et d'autre part l'accessibilité de la fonction phénol d'origine en position 7 est préservée.

Il est extrêmement important que cette fonction soit conservée dans le composé, monomère selon l'invention car elle est essentielle pour les mesures de pH ou pour générer des interactions par liaisons hydrogène avec d'autres substrats ou molécules pour des applications dans le domaine de la détection par exemple.

En conclusion selon l'invention, on fournit pour la première fois des composés de la coumarine modifiés chimiquement pour devenir directement et aisément polymérisables et qui, de manière étonnante, présentent toujours toutes les propriétés avantageuses de la coumarine de base telle que la 7-hydroxycoumarine.

L'invention concerne, en outre, un procédé de préparation de l'umbelliférone-4-acétate d'éthyl-2-méthacrylate, dans lequel on fait réagir l'acide umbelliférone-4-acétique avec un composé, qui est le méthacrylate de 2-hydroxyéthyle, comprenant d'une part une fonction hydroxy susceptible de réagir avec le groupement acide carboxylique de l'acide umbelliférone-4-acétique et d'autre part un groupement chimique polymérisable R qui est un groupement méthacrylate, moyennant quoi se produit un couplage du groupement acide carboxylique de l'acide umbelliférone-4-acétique avec la**dite** fonction **hydroxy** pour former l'umbelliférone-4-acétate d'éthyl-2-méthacrylate.

L'invention concerne également un polymère fluorescent susceptible d'être obtenu par polymérisation d'un composé monomère fluorescent polymérisable de la 7-hydroxycoumarine répondant à la formule (I) suivante : 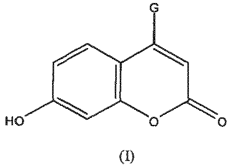 dans laquelle G est un substituant comprenant un groupement chimique polymérisable R ;
et éventuellement d'au moins un autre monomère polymérisable (IV).

Par groupement chimique polymérisable au sens de l'invention, on entend tout groupement susceptible de subir une réaction de polymérisation.

**Le** groupement chimique polymérisable R **est** choisi parmi les groupements vinyliques, styréniques, diéniques, acryliques, et méthacryliques.

Parmi les composés de formule (I), l'umbelliférone-4-acétate d'éthyl-2-méthacrylate est un composé nouveau.

Les composés de formule (I) peuvent être définis comme des dérivés fluorescents polymérisables, polaires, de la 7-hydroxycoumarine qui comportent en position 4, un substituant polymérisable qui ne modifie pas, ou qui ne modifie pas de manière substantielle les propriétés électroniques et la photoluminescence du noyau coumarine de base.

Les composés de formule (I) peuvent être considérés en particulier comme des dérivés de la 4-méthylumbelliférone encore appelée 7-hydroxy-4-méthylcoumarine.

Dans les composés de formule (I) d'une part les propriétés électroniques et de photoluminescence du fluorophore d'origine, à savoir la 7-hydroxycoumarine, sont conservées grâce à la fonctionnalisation du noyau coumarine spécifiquement en position 4, et d'autre part l'accessibilité de la fonction phénol d'origine en position 7 est préservée.

Il est extrêmement important que cette fonction soit conservée dans le composé, monomère de formule (I) car elle est essentielle pour les mesures de pH ou pour générer des interactions par liaisons hydrogène avec d'autres substrats ou molécules pour des applications dans le domaine de la détection par exemple.

En conclusion, les composés de formule (I) sont des composés de la coumarine modifiés chimiquement pour devenir directement et aisément polymérisables et qui, de manière étonnante, présentent toujours toutes les propriétés avantageuses de la coumarine de base telle que la 7-hydroxycoumarine.

Les composés de formule (I) peuvent être préparés par un procédé dans lequel on fait réagir un composé de formule (II) : 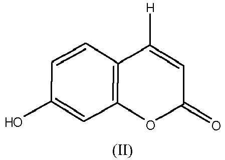 dans laquelle H est un substituant comprenant une fonction réactive A, avec un composé (III) comprenant d'une part une fonction réactive B susceptible de réagir avec la fonction réactive A et d'autre part, un groupement chimique polymérisable R, moyennant quoi se produit un couplage de la fonction réactive A avec la fonction réactive B pour former le substituant G comprenant le groupement chimique polymérisable R.

Ce procédé peut être défini comme un procédé de synthèse d'un monomère polymérisable fluorescent appartenant à la famille des coumarines.

Plus exactement, ce procédé consiste à coupler une coumarine (II) dont le substituant du noyau aromatique en position 4 comprend une fonction réactive A, avec un composé, que l'on peut appeler aussi monomère polymérisable comportant lui-même une fonction réactive B susceptible de réagir avec la fonction réactive A.

Le couplage des deux composés, entités (II) et (III) par l'intermédiaire des deux fonctions compatibles A et B se fait sans que les propriétés de la coumarine d'origine, notamment les propriétés d'émission de la coumarine d'origine ne se trouvent affectées et en laissant le groupement polymérisable R intact.

De plus, dans ce procédé, l'importante fonction -OH en position 7 du noyau coumarine n'est pas affectée par le couplage, tandis que la coumarine se trouve pourvue en position 4 (éloignée de la position 7) d'un groupement polymérisable R qui n'affecte pas les propriétés fondamentales du noyau coumarine et qui permet la polymérisation du composé fluorescent de manière facile.

Avantageusement, la fonction réactive A et la fonction réactive B peuvent être choisies parmi les groupements amine, hydrazine, hydrazone, azide, isocyanate, isothiocyanate, alcoxyamine, aldéhyde, époxy, nitrile, maléimide, halogéno, hydroxy, thiol, anhydride, acide carboxylique, chlorure d'acide tel que le chlorure d'acyle.

Bien entendu, A et B sont généralement choisies de manière à être compatibles, c'est-à-dire de manière à pouvoir réagir entre elles pour former une liaison covalente.

La fonction réactive A peut être directement reliée au noyau coumarine, ou bien elle peut être reliée au noyau coumarine par un bras espaceur tel qu'un groupe alkylène, de préférence un groupe alkylène de 1 à 10 atomes de carbone, de préférence encore de 1 à 4 atomes de carbone, qui peut être éventuellement interrompu par un ou plusieurs groupe(s) choisi(s) parmi -O- ; -CO- ; -S- ; et -N- .

L'ensemble de la fonction réactive A et du bras espaceur forme alors le substituant H du composé (II).

**Le** groupement chimique polymérisable R peut être choisi parmi les groupements vinyliques, styréniques, diéniques, acryliques, et méthacryliques.

Avantageusement, le composé (III) peut être choisi parmi les alkylacrylates, les alkylméthacrylates, les alkylacrylamides, les alkylméthacrylamides, les esters vinyliques, le styrène, et les diènes, comprenant une fonction réactive B.

En particulier, l'une parmi la fonction réactive A et la fonction réactive B peut être un groupement acide carboxylique, et l'autre parmi la fonction réactive A et la fonction réactive B peut être une fonction hydroxy et le couplage de la fonction réactive A avec la fonction réactive B peut se produire alors selon une réaction d'estérification.

Avantageusement, le composé (II) peut être l'acide umbelliférone-4-acétique, et le composé (III) peut être un composé avec une fonction réactive B qui est une fonction hydroxy tel que le méthacrylate de 2-hydroxyéthyle et l'on prépare ainsi le composé nouveau umbelliférone-4-acétate d'éthyl-2-méthacrylate.

Le polymère fluorescent selon l'invention comprend donc des motifs répétitifs issus du composé monomère fluorescent de formule (I) et éventuellement des motifs répétitifs issus d'au moins un autre monomère polymérisable (IV).

De même que dans le composé de formule (I), les propriétés de la coumarine d'origine, notamment les propriétés d'émission de la coumarine d'origine ne se trouvent pas affectées et sont intégralement conservées dans le polymère selon l'invention.

De plus, dans le polymère selon l'invention, l'importante fonction -OH en position 7 du noyau coumarine n'est pas affectée par la polymérisation et demeure libre, disponible.

Le polymère selon l'invention bénéficie donc de toutes les propriétés avantageuses du monomère de formule (I) dont il est issu.

Le polymère selon l'invention peut être un homopolymère susceptible d'être obtenu par polymérisation d'un composé monomère fluorescent de formule (I).

Un tel homopolymère n'a jamais été décrit ou suggéré dans l'art antérieur.

De préférence, cet homopolymère est susceptible d'être obtenu par polymérisation de l'umbelliférone-4-acétate d'éthyl-2-méthacrylate.

Ou bien, le polymère selon l'invention peut être un copolymère statistique, alterné ou séquencé (à blocs) susceptible d'être obtenu par polymérisation d'au moins un composé monomère fluorescent de formule (I) et d'au moins un autre monomère copolymérisable.

Avantageusement, cet autre monomère copolymérisable est choisi parmi les monomères acryliques, les monomères styréniques, et les monomères vinyliques.

Des copolymères préférés sont le copolymère, de préférence statistique, susceptible d'être obtenu par copolymérisation de l'umbelliférone-4-acétate d'éthyl-2-méthacrylate, du méthacrylate de 2-hydroxyéthyle, et du divinylbenzène, et le copolymère, de préférence statistique, susceptible d'être obtenu par copolymérisation de l'umbelliférone-4-acétate d'éthyl-2-méthacrylate, de la 4-vinylpyridine, et du divinylbenzène.

Le polymère selon l'invention peut avantageusement se présenter sous la forme de sphères, notamment de microsphères d'un diamètre de 0,1 à 500 µm, de préférence de 0,1 à 200 µm.

Le polymère selon l'invention peut se présenter sous la forme d'un film mince déposé sur au moins une surface d'un substrat, ou d'un film épais.

L'invention a trait, en outre, à un capteur comprenant le polymère selon l'invention en tant que matériau sensible.

La mise en oeuvre des polymères selon l'invention dans de tels capteurs n'a jamais été décrite ou suggérée dans l'art antérieur.

Avantageusement, ce capteur est un capteur optique dont le fonctionnement est basé sur la mesure des variations de l'intensité de la fluorescence émise par le polymère.

L'invention concerne en outre l'utilisation des capteurs tels que définis plus haut pour la détection et/ou le dosage d'un ou plusieurs composés, molécules, cibles.

De préférence, ce(ces) composé(s) est(sont) en phase vapeur, sous forme gazeuse.

Ces composés à détecter et/ou à doser peuvent être notamment choisis parmi les composés nitrés et les composés organophosphorés.

Les composés nitrés peuvent être choisis parmi les composés nitroaromatiques, les nitramines, les nitrosamines et les esters nitriques.

De manière préférée, le polymère fluorescent selon l'invention et donc le capteur comprenant ce polymère peuvent être utilisés pour la détection et/ou le dosage d'explosifs ou de composés, notamment de gaz, toxiques en particulier neurotoxiques.

L'invention concerne enfin l'utilisation des polymères selon l'invention pour la réalisation de sondes fluorescentes, pour la réalisation de marqueurs biologiques, pour la réalisation de matériaux fluorescents, photoluminescents et/ou radioluminescents et/ou cathodoluminescents.

D'autres caractéristiques et avantages de l'invention apparaitront mieux à la lecture de la description qui suit, donnée à titre illustratif et non limitatif, et qui est faite en référence aux dessins joints dans lesquels :

### BRÈVE DESCRIPTION DES DESSINS

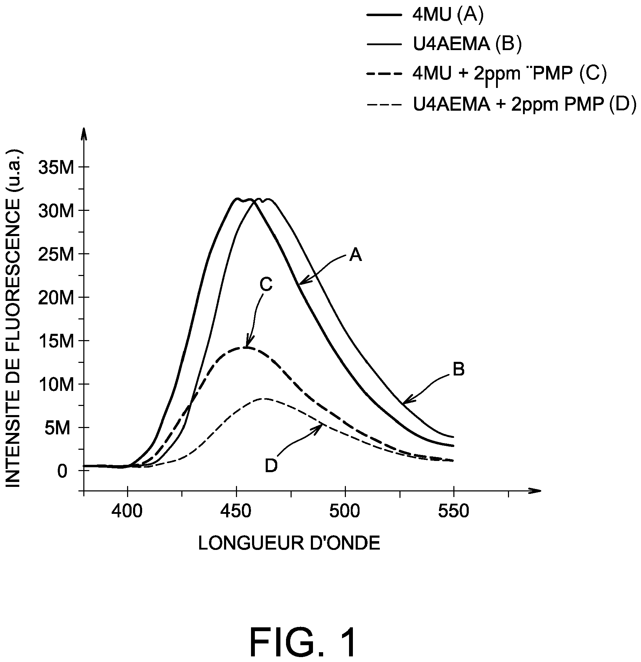

- La figure 1 est un graphique qui montre les spectres d'émission de solutions de 4MU (courbe A), et de U4AEMA (courbe B) à 0,01 g dans l'éthanol pur, d'une solution de 4MU à 0,01 g/L dans l'éthanol pur avec addition de 2 ppm de PMP (courbe C), et d'une solution de U4AEMA à 0,01 g/L dans l'éthanol pur avec addition de 2 ppm de PMP (courbe D), pour une longueur d'onde d'excitation λₓ de 370 nm. Les spectres de la 4MU et de U4AEMA ont été normalisés à la même intensité.

En ordonnée est portée l'intensité de fluorescence (en u.a.), et en abscisse est portée la longueur d'onde (en nm).

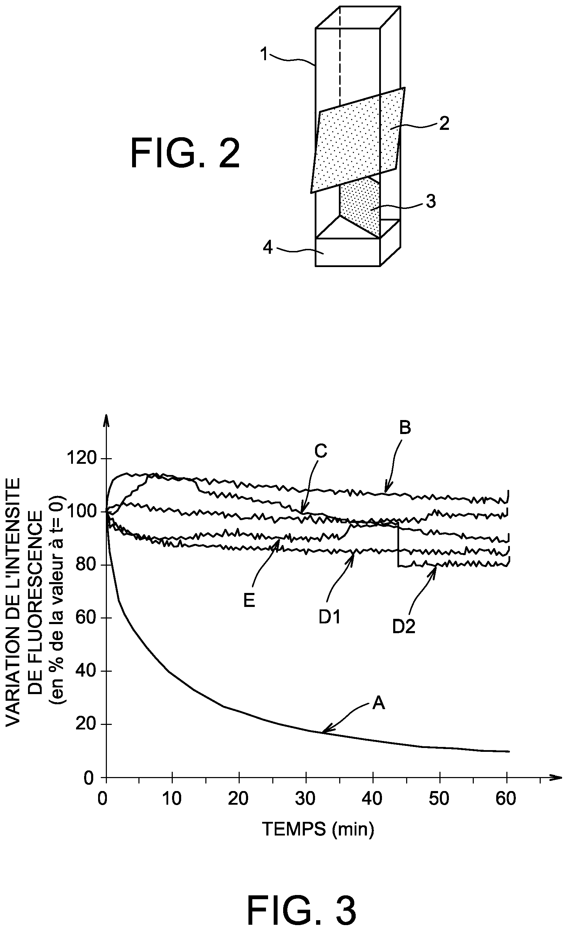

- La figure 2 est une vue en perspective d'une cuve en quartz pour mesure de fluorescence montée pour une mesure en atmosphère statique à la pression de vapeur saturante du nitrobenzène.
- La figure 3 est un graphique qui donne la variation de l'intensité de fluorescence de dépôts d'un copolymère fluorescent à base d'umbelliférone-4-acétate d'éthyl-2-méthacrylate, de 4-vinylpyridine et de divinylbenzène, en présence de vapeurs statiques de nitrobenzène (Courbe A), d'éthanol (Courbe B), de toluène (Courbe C), d'acétone (Courbes Dl et D2), et de Méthyl-Ethyl-Cétone (MEK) (Courbe E) (Longueur d'onde d'excitation λₓ = 320 nm, Longueur d'onde d'émission λ_{M} = 390 nm, fentes = 0,1 nm, fréquence = 10 s⁻¹).

En ordonnée est portée la variation de l'intensité de fluorescence (en % de la valeur à t = O), et en abscisse est porté le temps (en minutes).

### EXPOSÉ DÉTAILLÉ DE MODES DE RÉALISATION PARTICULIERS

Les composés ou monomères fluorescents, polymérisables, de la 7-hydroxycoumarine qui peuvent être utilisés pour préparer les polymères selon l'invention répondent à la formule (I) suivante : 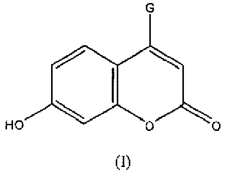 dans laquelle G est un substituant comprenant un groupement chimique polymérisable R.
R est généralement un groupement comprenant une double liaison carbone - carbone éthyléniquement insaturée.

D'autres groupements chimiques polymérisables sont les groupements permettant des réactions de condensation ou les groupements permettant des réactions de couplage organométallique ou oxydant.

Parmi les groupements R comprenant une double liaison carbone - carbone éthyléniquement insaturée, on peut citer les groupements vinyliques, styréniques, diéniques, acryliques, et méthacryliques.

Le groupement R peut être relié directement au noyau coumarine en position 4 de celui-ci ou bien il peut être relié au noyau coumarine par l'intermédiaire d'un groupe de liaison tel qu'un groupe alkylène, comprenant généralement de 1 à 10 C, de préférence de 1 à 4 C, qui peut être éventuellement interrompu par un ou plusieurs groupes choisis parmi -O- ; -CO- ; -S- ; et -N- .

Parmi ces composés fluorescents, certains sont nouveau, dont en particulier l'umbelliférone-4-acétate d'éthyl-2-méthacrylate.

L'invention concerne donc aussi l'umbelliférone-4-acétate d'éthyl-2-méthacrylate à titre de composé nouveau.

Le procédé de préparation des composés de formule (I) consiste à coupler selon le schéma 1 une 7-hydroxycoumarine dont le substituant du noyau aromatique en position 4 comprend une fonction réactive A, avec un monomère polymérisable comprenant un groupement polymérisable R et une fonction réactive B, susceptible de réagir avec la fonction réactive A.

Le couplage de ces deux entités, à savoir d'une part la 7-hydroxycoumarine et d'autre part le monomère polymérisable n'affecte pas les propriétés d'émission de la 7-hydroxycoumarine de départ.

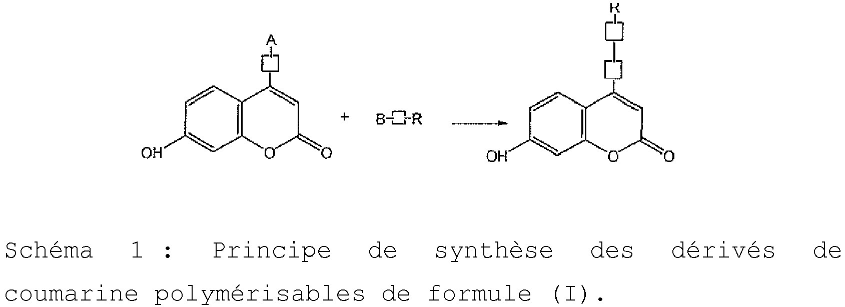

La fonction réactive A et la fonction réactive B peuvent être choisies parmi les groupements amine, hydrazine, hydrazone, azide, isocyanate, isothiocyanate, alcoxyamine, aldéhyde, époxy, nitrile, maléimide, halogèno, hydroxy, thiol, anhydride, acide carboxylique, chlorure d'acide tel que chlorure d'acyle.

Dans le schéma 2, R représente tout type de groupement chimique polymérisable, tel que défini plus haut.

Tout monomère comprenant au moins une fonction réactive B capable de réagir avec la fonction réactive A, et un groupement polymérisable R autre que ladite fonction réactive B peut convenir pour réaliser la synthèse d'un composé polymérisable de formule (I) par le procédé exposé plus haut.

Dans ce monomère polymérisable, la fonction réactive B et le groupement polymérisable R peuvent être directement reliés mais ils sont généralement séparés par un groupe de liaison tel qu'un groupe alkylène, comprenant généralement de 1 à 10 C, de préférence de 1 à 4 C, qui peut être éventuellement interrompu par un ou plusieurs groupes choisis parmi -O- ; -CO ; -S- ; et -N- .

Un monomère particulièrement adapté, dont la structure est représentée ci-dessous (formule (2)) est le méthacrylate de 2-hydroxyéthyle qui possède en tant que fonction réactive B, une fonction alcool réactive et en tant que groupement polymérisable R, un groupement méthacryle, R et B étant reliés par une chaîne -(CO)-O-(CH₂)₂-OH.

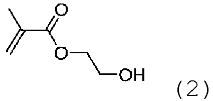

D'autres exemples non limitatifs de monomères appropriés pour la synthèse du composé, monomère, fluorescent polymérisable de formule (I) sont les dérivés fonctionnels de monomères tels que les alkylacrylates, les alkylméthacrylates, les alkylacrylamides, les alkylméthacrylamides, les esters vinyliques, les styrènes et les diènes. Par dérivés fonctionnels, on entend que ces composés outre un groupement polymérisable tel qu'un groupement méthacryle, acryle, vinyle comprennent une fonction réactive B telle que définie plus haut.

L'homme du métier saura choisir parmi les fonctions A et les fonctions B celles qui peuvent donner les réactions de couplage adéquates et il saura facilement déterminer les conditions de ces réactions, notamment afin que le groupe hydroxy en position 7 ne soit pas affecté.

En particulier, la réaction de couplage entre la fonction réactive A et la fonction réactive B peut être une réaction d'estérification entre un alcool et un acide carboxylique, l'acide carboxylique pouvant être indifféremment la fonction réactive A ou la fonction réactive B.

Selon un mode de réalisation préféré de l'invention, on pourra donc partir de l'acide umbelliférone-4-acétique, qui est un dérivé de la 7-hydroxycoumarine substituée en position 4 par un groupement acide acétique et qui répond à la formule (3) suivante :

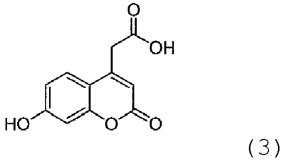

La préparation du dérivé de la 7-hydroxycoumarine, fluorescent substitué en position 4 par un groupement polymérisable est effectuée selon le schéma de synthèse 2.

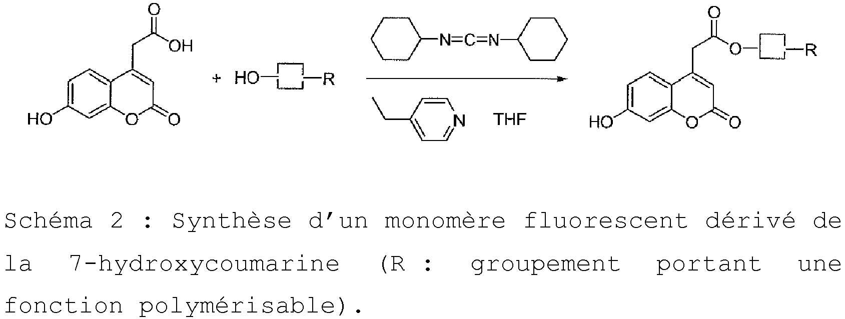

Le couplage se fait selon une réaction d'estérification entre le composé représenté sur la figure 4 et un alcool portant un groupement polymérisable R en présence de dicyclohéxylcarbodiimide et d'une base azotée telle que l'éthylpyridine dans un solvant tel que le THF.

On obtient ainsi un monomère fluorescent polymérisable, dérivé de la 7-hydroxycoumarine, qui porte une fonction polymérisable R comprenant par exemple une double liaison carbone-carbone éthyléniquement insaturée.

Un composé, monomère, fluorescent, polymérisable préféré nouveau selon l'invention dont on peut réaliser la synthèse par le procédé décrit plus haut est l'umbelliférone-4-acétate d'éthyl-2-méthacrylate de formule (4).

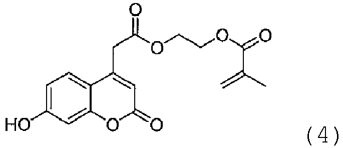

Ce composé peut être préparé par une réaction d'estérification du composé de formule (3) avec le méthacrylate de 2-hydroxyéthyle représenté par la formule (2).

Selon l'invention, des polymères, homopolymères et copolymères peuvent être préparés par polymérisation des monomères fluorescents polymérisables de formule (I) décrits plus haut et dont la synthèse a également été décrite ci-dessus.

Le polymère selon l'invention peut être un homopolymère, c'est-à-dire qu'il est préparé à partir d'un seul composé, monomère de formule (I). En d'autres termes, il est constitué par un seul motif répétitif issu d'un composé, monomère de formule (I).

Un tel homopolymère peut être préparé par tout procédé de polymérisation connu. Toutefois, de préférence, cet homopolymère est préparé par un procédé de polymérisation radicalaire, contrôlée ou non.

Un homopolymère selon l'invention a généralement un degré de polymérisation n de 1 à 10000, de préférence de 5 à 100.

En particulier, l'invention concerne les homopolymères susceptibles d'être préparés par polymérisation du composé de formule (6) l'umbelliférone-4-acétate d'éthyl-2-méthacrylate.

Ces homopolymères sont représentés par la formule (5) : 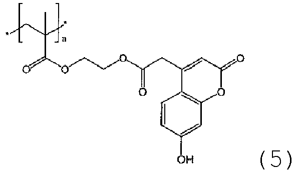 dans laquelle n est un nombre entier de 1 à 10000, de préférence de 5 à 100.

Il a été mis en évidence que l'homopolymère de la formule (5), préparé à partir de l'umbelliférone-4-acétate d'éthyl-2-méthacrylate présente de manière inattendue des propriétés singulières.

En effet, il a été constaté que l'homopolymère correspondant à un degré de polymérisation de n∼120, présente une transition en température de type LCST (« Lower Critical Solution Temperature » en anglais) lorsqu'il est solubilisé dans le tétrahydrofuranne (THF).

Ainsi, à température ambiante, la solution de polymère dans le THF est monophasique. Lorsque la solution est chauffée, on observe une séparation de phase et un mélange biphasique. Le phénomène est parfaitement réversible et on récupère une solution limpide si on laisse l'échantillon revenir à temperature ambiante.

Le groupe pendant coumarinyl peut en effet former une liaison H « intramotif » (Formule 6) ou « intermotif/interchaîne » (Formule 7) pouvant justifier l'existence de cette transition. Cette propriété originale pourrait ainsi être exploitée pour fabriquer des thermomètres fluorescents par exemple.

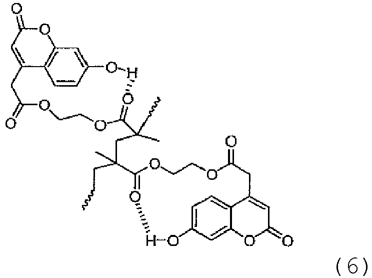

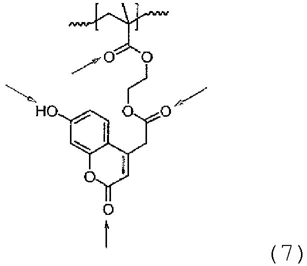

Par conséquent, une autre innovation intéressante liée à la synthèse de ce type de monomère fluorescent est la possibilité de former une liaison hydrogène intramoléculaire, conférant des propriétés singulières aux polymères qui en dérivent.

En variante, le polymère selon l'invention peut être un copolymère c'est-à-dire qu'il est alors préparé par polymérisation de plusieurs monomères selon l'invention qui répondent tous à la formule (I), ou par polymérisation d'un ou de plusieurs monomères de formule (I) selon l'invention et d'un ou de plusieurs autres monomères polymérisables.

Ce ou ces autre(s) monomère(s) copolymérisable(s) peut (peuvent) être choisi(s) parmi les monomères (méth)acryliques, les monomères styréniques, et les monomères vinyliques.

Des exemples de ces autres monomères copolymérisables sont l'acrylate de méthyle, le méthacrylate de méthyle, le cyanoéthylacrylate, le méthacrylate de 2-hydroxyéthyle, le divinylbenzène, la 4-vinylpyridine.

Les copolymères fluorescents selon l'invention peuvent être des copolymères statistiques, alternés ou séquencés (à blocs) selon les rapports de réactivité des comonomères.

Des copolymères préférés sont les copolymères susceptibles d'être obtenus par copolymérisation de l'umbelliférone-4-acétate d'éthyl-2-méthacrylate, du méthacrylate de 2-hydroxyéthyle, et du divinylbenzène, et les copolymères susceptibles d'être obtenus par copolymérisation de l'umbelliférone-4-acétate d'éthyl-2-méthacrylate, de la 4-vinylpyridine, et du divinylbenzène.

Dans les copolymères selon l'invention préparés à partir d'un monomère fluorescent de formule (I) selon l'invention et d'un autre monomère polymérisable, le degré de polymérisation n du monomère fluorescent de formule (I) selon l'invention est généralement de 1 à 10000, de préférence de 5 à 100, et le degré de polymérisation n' de l'autre monomère est généralement de 1 à 10000, de préférence de 5 à 100.

La formule (8) représente à titre d'exemple un copolymère fluorescent issu de la copolymérisation d'un monomère fluorescent polymérisable de formule (I) selon l'invention, à savoir l'umbelliférone-4-acétate d'éthyl-2-méthacrylate et d'un autre monomère polymérisable P.

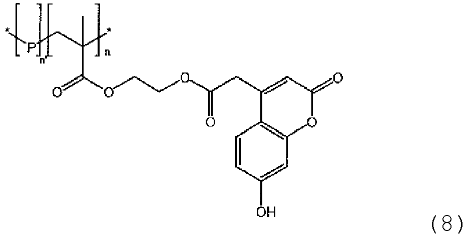

Le copolymère représenté sur cette formule (8) où n et n' sont tels que définis plus haut, peut être aussi bien un copolymère statistique, qu'un copolymère alterné, qu'un copolymère séquencé.

En outre, les copolymères selon l'invention peuvent être hydrosolubles ou bien solubles dans les solvants organiques tandis que les homopolymères sont généralement seulement organosolubles. Cette solubilité des polymères selon l'invention s'avère particulièrement intéressante pour préparer des films minces à partir de ces polymères.

Leur solubilité notamment dans l'eau peut être facilement contrôlée par l'homme du métier en ajustant la nature et la proportion de chacun des comonomères. Ainsi, si l'on souhaite que le copolymère soit soluble dans l'eau, l'autre monomère polymérisable sera-t-il choisi parmi les monomères hydrophiles connus de l'homme du métier dans une proportion telle que la solubilité recherchée soit obtenue.

Les copolymères selon l'invention peuvent être préparés par tout procédé de polymérisation connu. Toutefois, de préférence, ces copolymères sont préparés par un procédé de polymérisation radicalaire, contrôlée ou non. Comme on l'a précisé plus haut selon le rapport de réactivité des comonomères, les copolymères obtenus pourront être statistiques, alternés ou à blocs et la solubilité des polymères pourra être contrôlée par l'homme du métier en choisissant de manière adéquate la nature et la proportion de chacun des comonomères.

Le polymère selon l'invention peut se présenter sous une forme quelconque.

Le polymère selon l'invention peut avantageusement se présenter sous la forme de sphères, notamment de microsphères d'un diamètre de 0,1 à 500 µm, de préférence de 0,1 à 200 µm.

Les polymères selon l'invention peuvent se présenter sous la forme d'un monolithe, par exemple d'un cylindre présentant de préférence une certaine porosité de manière à rendre accessible la totalité du polymère à des composés à détecter ou à doser.

Les polymères selon l'invention peuvent aussi se présenter sous la forme de films.

Ces films peuvent être des films minces, généralement déposés sur au moins une surface d'un substrat. Dans le cas d'un substrat plan, ce film mince peut recouvrir l'une ou les deux faces du substrat.

Lorsque le polymère selon l'invention se présente sous la forme d'un film mince, celui-ci a généralement une épaisseur de 10 Angstrôms à 100 microns.

Un tel film peut être préparé par tout procédé connu de l'homme du métier pour préparer un film mince sur la surface d'un substrat par exemple :
- par pulvérisation, enduction centrifuge (« spin coating »), ou dépôt à la goutte (« drop coating ») ;
- par trempage-retrait (« dip-coating ») ;
- par la technique de Langmuir-Blodgett ;
- par dépôt électrochimique ;
- par polymérisation in-situ, c'est-à-dire par directement sur la surface du substrat.

Les polymères selon l'invention présentent généralement une solubilité dans les solvants organiques tels que l'acétone, le chloroforme, le THF, le DMF, le DMSO, la NMP, qui les rend particulièrement aptes à leur mise en oeuvre dans des procédés de préparation de couches minces en solution.

Le polymère selon l'invention peut aussi se présenter sous la forme d'un film épais, généralement autosupporté.

Un tel film épais a généralement une épaisseur de 100 µm jusqu'à un ou plusieurs mm (par exemple, 2, 3, 4, 5 mm) et peut être préparé par pulvérisation, polymérisation in-situ, étalement à la main (« hand coating »), pressage à chaud, extrusion.

L'invention a trait, en outre, à des capteurs comprenant ledit polymère sous toute forme que ce soit en tant que matériau sensible.

Ces capteurs comprenant par exemple le polymère sous la forme d'une couche mince ou bien de microsphères peuvent être notamment des capteurs optiques dont le fonctionnement est basé sur la mesure des variations de l'intensité de la fluorescence émise par le polymère.

Le principe de fonctionnement des capteurs optiques à base de fluorescence a notamment été décrit par B. Valeur dans Molecular Fluorescence : Principles and Applications, 2002, Ed. WILEY VCH, New York. Généralement, ces capteurs comprennent un substrat en verre de qualité optique dont l'une des faces est recouverte d'un film mince du matériau sensible. L'intensité de la fluorescence émise par le matériau sensible peut être mesurée sur l'ensemble du spectre d'émission de ce matériau. Toutefois, il est préférable d'effectuer les mesures d'intensité de fluorescence à la longueur d'onde d'émission donnant les valeurs d'intensité maximales pour la longueur d'onde d'excitation conduisant, elle, au meilleur rapport signal/bruit pour l'acquisition des intensités de fluorescence.

Des capteurs comportant un polymère tel que précédemment défini, en tant que matériau sensible, se sont révélés présenter de nombreux avantages, notamment :
* une aptitude à détecter spécifiquement les composés nitrés, et en particulier les composés nitroaromatiques ainsi que les composés organophosphorés, avec une grande sensibilité puisqu'ils sont capables de détecter leur présence à des concentrations inférieures au ppm (partie par million) et même au dixième de ppm, doublée d'une spécificité vis-à-vis de ces composés,
* une rapidité de réponse et une reproductibilité de cette réponse,
* une stabilité des performances dans le temps et, partant, une durée de vie très satisfaisante,
* une aptitude à fonctionner en continu,
* un coût de fabrication compatible avec une production de capteurs en série, une très faible quantité de polymère (c'est-à-dire en pratique de quelques mg) étant nécessaire pour la fabrication d'un capteur, et
* la possibilité d'être miniaturisés et, partant, d'être aisément transportables et manipulables sur tout type de sites.

Ces capteurs peuvent être notamment utilisés pour la détection et/ou le dosage d'un ou plusieurs composés, molécules, cibles.

Ces composés cibles peuvent se présenter sous forme solide, liquide ou gazeuse (vapeur) mais de préférence ces composés sont en phase vapeur, sous forme gazeuse.

En d'autres termes dans le domaine de la détection chimique, les polymères fluorescents selon l'invention se sont révélés capables de signaler la présence de molécules cibles en phase vapeur. La détection repose sur la mesure de variations de fluorescence du polymère, par exemple sous la forme d'un film, lorsqu'il est exposé à des vapeurs de la molécule cible.

La qualité définie par la sensibilité, la sélectivité et la réversibilité de la détection est en grande partie conditionnée par les propriétés de la couche sensible et il s'est avéré que les polymères fluorescents selon l'invention possèdent des propriétés de photoluminescence particulièrement intéressantes pour ce type d'application en vue de la détection et/ou du dosage de composés.

Les composés à détecter et/ou à doser peuvent être notamment choisis parmi les composés nitrés et les composés organophosphorés.

Les composés nitrés peuvent être choisis parmi les composés nitroaromatiques, les nitramines, les nitrosamines et les esters nitriques.

A titre d'exemples de composés nitroaromatiques, on peut citer le nitrobenzène, le dinitrobenzène, le trinitrobenzène, le nitrotoluène, le dinitrotoluène, le trinitrotoluène, le dinitrofluoro-benzène, le dinitrotrifluorométhoxybenzène, l'amino-dinitrotoluène, le dinitrotrifluorométhylbenzène, le chlorodinitrotrifluorométhylbenzène, l'hexanitro-stilbène ou encore le trinitrophénol (ou acide picrique).

A titre d'exemples de nitramines, on peut citer la cyclotétraméthylènetétranitramine (ou octogène), la cyclotriméthylènetrinitramine (ou hexogène) et la trinitrophénylméthylnitramine (ou tétryle), tandis que les nitrosamines sont, par exemple, la nitrosodiméthylamine.

A titre d'exemple d'esters nitriques, on peut citer la pentrite, le dinitrate d'éthylène glycol, le dinitrate de diéthylène glycol, la nitroglycérine ou la nitroguanidine.

A titre d'exemples de composés organophosphorés, on peut citer le Sarin, le VX, le Tabun, le Soman, le Cyclosarin, le diisopropyl fluoro phosphonate (DFP), l'Amiton ou VG, le Parathion, le Diméthoxy méthylphosphonate (DMMP).

De manière préférée, le polymère fluorescent selon l'invention et donc le capteur comprenant ce polymère peuvent être utilisés pour la détection et/ou le dosage d'explosifs ou de composés, notamment de gaz, toxiques en particulier neurotoxiques, y compris les pesticides organophosphorés.

A titre d'exemple, un film du polymère obtenu par copolymérisation du monomère fluorescent umbelliférone-4-acétate d'éthyl-2- méthacrylate, de 2-hydroxyéthylméthacrylate et de divinylbenzène (voir exemple 5), présente de façon inattendue une augmentation de son intensité de fluorescence à 390 nm en présence de vapeurs de diéthylchlorophosphate (Formule 9), un simulant des gaz neurotoxiques organophosphorés. Par ailleurs, un film d'un polymère obtenu par copolymérisation du monomère fluorescent umbelliferone-4-acétate d'éthyl-2-méthacrylate, de 4-vinylpyridine et de divinylbenzène, a présenté une atténuation de fluorescence à 390 nm en présence de vapeurs de nitrobenzène (Formule 10), un simulant des explosifs nitroaromatiques (voir exemple 5).

Ainsi, les polymères fluorescents de cette invention présentent des propriétés d'émission de fluorescence exploitables pour la détection chimique.

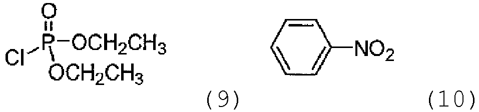

Outre leur utilisation comme matériau sensible de capteurs, les polymères selon l'invention peuvent être utilisés pour la réalisation de sondes fluorescentes, pour la réalisation de marqueurs biologiques, pour la réalisation de matériaux fluorescents, photoluminescents et/ou radioluminescents et/ou cathodoluminescents. De tels matériaux trouvent notamment leur application dans la réalisation de dispositifs d'imagerie et de lasers organiques tout-solide.

Ainsi, dans un dispositif d'imagerie, le polymère fluorescent déposé sur un substrat, capte des photons, électrons, radiations... qui le font fluorescer. La lumière émise est récupérée par l'intermédiaire d'une caméra CCD qui permet de faire une analyse spatiotemporelle.

Dans un laser organique solide, la source fluorescente liquide telle que de la coumarine en solution est remplacée par le polymère.

L'invention va maintenant être décrite en référence aux exemples suivants donnés à titre illustratif et non limitatif.

### Exemple 1 : Synthèse du monomère umbelliférone-4-acétate d'éthyl-2-méthacrylate (U4AEMA).

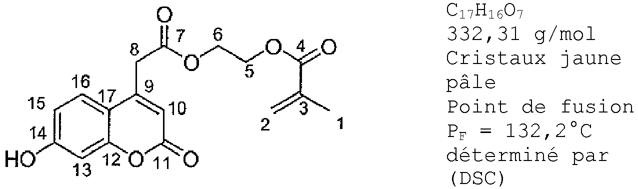

Dans un ballon, 1 g (4,5 mmoles) d'acide umbelliférone-4-acétique et 40 mL de tétrahydrofuranne anhydre non stabilise sont introduits, donnant une solution limpide jaune. Puis, 0,48 g (4,5 mmoles) d'éthylpyridine, 2,96 g (22,5 mmoles) de 2-hydroxyéthylmethacrylate et 0,94 g (4,5 mmoles) de dicyclohexylcarbodiimide préalablement dissous dans 35 mL de tétrahydrofuranne anhydre non stabilisé, sont ajoutés à la solution à l'abri de la lumière. Le mélange est agité à temperature ambiante pendant 5 heures. Après élimination du précipité de dicyclohexylurée par filtration, le solvant est évaporé à l'abri de la lumière. Le produit est purifié sur colonne de gel de silice éluée avec un mélange heptane/acétone (3:1, v/v). Après évaporation du solvant, le produit pur est obtenu sous forme d'un solide jaune pâle. Après séchage, un rendement de 68% est obtenu.

| | |
|---|---|
| **RMN ¹H** (200 MHz, CDCl₃) δ (ppm) : | 1.91 (3H, t, J=1.5 Hz, **-CH₃: H¹**), 3.78 (2H, s, **CH₂**COO, **H⁸**), 4.40 (4H, m, O(**CH**₂)₂O : **H^{5,6}**), 5.59 (1H, m, **=CH₂ trans, H²**), 6.06 (1H, m, **=CH₂ cis, H²**), 6.24 (1H, s, **H¹⁰**), 6.83 (1H, dd, J=2.4/8.6 Hz, **H¹⁵**), 6.89 (1H, d, J=2.4 Hz, **H¹³**),6.99 (1H, s, **OH**)**,** 7.46 (1H, d, J=8.6 Hz, **H¹⁶**). |
| **RMN ¹³C** (200 MHz, CDCl₃) δ (ppm) : | 18.19 (**C¹**), 38.06 (**C⁸**), 63.35 (**C^{5.6}**), 103.58 (**C¹³**), 112.28 (**C¹⁰**), 113.53 (**C¹⁵**), 125.87 (**C²**), 126.45 (**C¹⁶**), 136.22 (**C³**), 148.27 (**C¹⁴**), 154.83 (**C⁹**), 155.62 (**C¹⁷**), 160.00 (**C¹¹**), 161.57 (**C¹²**), 167.51 (**C⁴**), 168.69 (**C⁷**). |
| **IR** (pastille KBr) v (cm⁻¹) : | 3210 (m), 2959 (f), 1744 (F), 1704 (F), 1686 (F), 1621 (F), 1609 (F), 1566 (F), 1510 (f), 1453 (f), 1403 (m), 1374 (m), 1341 (m), 1321 (F), 1277 (m), 1235 (f), 1208 (f), 1185 (m), 1157 (F), 1141 (m), 1068 (f), 1052 (f), 1035 (f), 962 (f), 950 (f), 935 (f), 894 (f), 876 (f), 843 (f), 821 (f), 744 (f), 717 (f), 698 (f), 653 (f), 610 (f), 593 (f), 484 (f), 455 (f). |
| **Fluorescence** (EtOH) λₘₐₓ (nm) | 464 (λₓ=370). |

Afin de vérifier que la fonctionnalisation du fluorophore est effectivement sans conséquence sur ses propriétés de fluorescence, nous avons comparé les spectres d'émission de solutions de 4-méthylumbelliférone (4MU) et d'umbelliferone-4-acétate d'ethyl-2-methacrylate (U4AEMA) dans l'éthanol (0,01 g/L). La figure 1 présente les résultats obtenus ainsi que l'effet de l'ajout d'une solution d'un acide phosphonique (le pinacolyl méthylphosphonate ou PMP, 2 ppm).

La figure 1 montre que la fonctionnalisation de la 4MU se traduit sur le spectre d'émission par un décalage bathochrome de 10 nm montrant ainsi que ses propriétés électroniques n'ont pas été modifiées de façon appréciable. Le fluorophore ainsi fonctionnalisé reste toujours sensible à la présence d'un acide dans le milieu et subit une atténuation de fluorescence à 460 nm.

### Exemple 2 : Synthèse de l'homopolymère poly(umbelliférone-4-acétate d'éthyl-2-méthacrylate)(poly(U4AEMA))

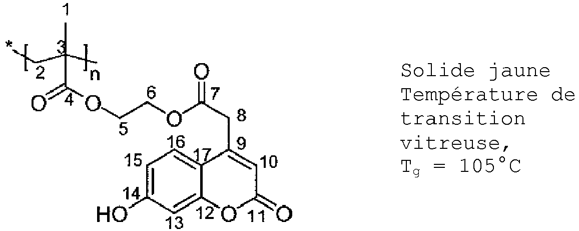

Dans un ballon tricol, 300 mg (0,9 mmoles) de U4AEMA et 6 mg (∼2% en poids) d'azobisisobutyronitrile (AIBN) comme amorceur, sont dissous dans 12 mL de tétrahydrofuranne (THF) anhydre non stabilisé, donnant une solution limpide jaune. Le mélange est placé 5 minutes au bain à ultrasons à l'abri de la lumière. Le ballon est ensuite surmonté d'un réfrigérant et placé dans un bain de glace alors qu'un bullage d'argon est effectué pendant 10 minutes dans la solution. Le mélange réactionnel est chauffé à 60°C pendant 20 heures sous agitation magnétique et léger balayage d'argon.

Le polymère formé est précipité dans 200 mL d'éther. Le précipité est ensuite filtré puis rincé. Le solide obtenu est repris dans le THF, puis à nouveau précipité dans l'éther. Après filtration et rinçage, le solide est séché jusqu'à masse constante. Le polymère est récupéré sous forme d'un solide jaune fluorescent avec un rendement de 52%.

| | |
|---|---|
| **GPC** (étalons PS dans le DMF) | M_{w} = 107300; Mₙ =38900 ; M_{w}/Mₙ =2.8 |
| **RMN ¹H** (200 MHz, CDCl₃) δ (ppm) : | 0.84 (5H, **H^{1,2}**), 3.81 (2H, **H⁸**), 4.17 (4H, **H^{5,6}**), 6.11 (1H, **H¹⁰**), 6.61 (2H, **H^{13,15}**), 7.37 (1H, **H¹⁶**), 10.54 (1H, **OH**). |
| **IR** (pastille KBr) v (cm⁻¹) : | 3362 (m), 2944 (m), 2803 (f), 2584 (f), 1720 (F), 1609 (F), 1566 (m), 1515 (f), 1445 (m), 1394 (m), 1317 (m), 1267 (m), 1237 (m), 1148 (F), 1138 (F), 1053 (m), 998 (m), 947 (f), 847 (m), 817 (f), 742 (f), 700 (f), 634 (f), 600 (f), 586 (f). |
| **Fluorescence** (film sur quartz) λₘₐₓ (nm) | 435 (λₓ=375). |

### Exemple 3 : Synthèse du copolymère poly(umbelliférone-4-acétate d'éthyl-2-méthacrylate-co-styrène).

Dans un ballon tricol parfaitement sec surmonté d'un réfrigérant, 5 mg (15 µmoles) de U4AEMA et 20 mg (∼2% en poids) d'AIBN sont dissous dans 20 mL de tétrahydrofuranne (THF) anhydre non stabilisé, introduit sous argon. Puis, 1 g (9,6 mmoles) de styrène purifié est ajouté sous argon. Le ballon est ensuite placé dans un bain de glace alors qu'un bullage d'argon est effectué pendant 20 minutes dans la solution. Le mélange réactionnel sous atmosphère inerte est chauffé à 60°C pendant 40 heures sous agitation magnétique. Le polymère formé est précipité dans 200 mL de méthanol. Le précipité est ensuite filtré puis rincé. Le solide obtenu est repris dans le THF, puis à nouveau précipité dans le méthanol. Après filtration et rinçage, le solide est séché à masse constante. Le polymère est récupéré sous forme d'un solide blanc fluorescent avec un rendement de 2%.

| | |
|---|---|
| **GPC** (étalons PS dans le THF) | M_{w}=6437 ; Mₙ=5028 ; M_{w}/Mₙ=1.28 |
| **IR** (pastille KBr) v (cm⁻¹) : | 3433 (f), 3100 (f), 3081 (m), 3060 (m), 3025 (F), 3000 (f), 2922 (F), 2847 (m), 1941 (f), 1869 (f), 1800 (f), 1731 (f), 1600 (m), 1582 (f), 1492 (F), 1452 (F), 1368 (f), 1324 (f), 1260 (f), 1180 (f), 1154 (f), 1066 (m), 1028 (m), 976 (f), 961 (f), 938 (f), 904 (f), 837 (f), 801 (f), 756 (F), 700 (F), 668 (f), 538 (m). |
| **Fluorescence** (film sur quartz) λₘₐₓ (nm) | 439 (λₓ=375). |

### Exemple 4 : Synthèse par précipitation de microsphères fluorescentes par copolymérisation de l'umbelliférone-4-acétate-2-méthacrylate, du divinylbenzène et du 2-hydroxyéthyl méthacrylate.

Dans un ballon monocol, un mélange acétonitrile/toluène (3:1, v/v) est introduit. Le 2-hydroxyéthyl méthacrylate et le divinylbenzène sont ensuite introduits dans un rapport molaire de 1:5 respectivement et de sorte que le mélange de solvants représente un pourcentage volumique de 96,5%. Le monomère fluorescent est ajouté à raison de 1 mol% par rapport aux autres monomères. Une fois l'amorceur (AIBN, 2% en poids) additionné, le mélange de synthèse est placé pendant 5 minutes dans un bain à ultrasons. Un bullage d'argon de 10 minutes suivi d'un balayage de 5 minutes sont opérés alors que le mélange est maintenu dans un bain de glace. Le ballon est ensuite fixé sur un bras rotatif permettant de faire tourner le ballon suivant un axe à 45°C environ, tout en le plongeant dans un bain d'huile. Une rampe de temperature est alors appliquée de façon à amener la temperature du bain d'huile de 20 à 60°C en l'espace de 2 heures. Le milieu réactionnel est ensuite chauffé à 60°C pendant 18 heures avec une rotation du ballon à 10 tr/min.

Les particules sphériques formées sont récupérées soit par filtration sous vide sur membrane millipore de 0,45 µm, soit par centrifugation à 11000 tr/min pendant 10 minutes (centrifugeuse Sigma 6K15, Fischer Bioblock Scientific).

L'extraction des monomères n'ayant pas réagi est opérée par extraction Soxhlet à l'aide d'un mélange méthanolfacide acétique (9:1, v/v) pendant une nuit (avec une vidange de l'extracteur toutes les 20 minutes environ). Puis les matériaux sont rincés au méthanol jusqu'à atteindre un pH neutre. Un dernier lavage à l'acétone est réalisé, avant séchage pendant une nuit à 40°C sous vide dynamique.

On obtient finalement une poudre blanche (rendement 25%) constituée de particules de 2 µm de diamètre.

### Exemple 5 :

Des microsphères fluorescentes sont synthétisées suivant le protocole décrit dans l'exemple 4, en remplaçant simplement le 2-hydroxyéthylméthacrylate par la 4-vinylpyridine.

Un dépôt de ces microsphères fluorescentes est réalisé sur un adhésif double face qui est placé sur un substrat en quartz (2) avant d'être introduit dans une cuve en quartz pour fluorescence (1) contenant du nitrobenzène liquide (4), comme schématisé sur la figure 2. Le substrat en quartz (2) est supporté par un porte-échantillon (3). La cuve fermée est ainsi à la pression de vapeur saturante du nitrobenzène (180 ppm).

La figure 3 représente l'évolution de l'intensité de fluorescence des microsphères au cours du temps, mesurée à 390 nm pour une longueur d'onde d'excitation de 320 nm, et ce dès l'introduction de l'échantillon dans la cuve. La même expérience est ensuite réalisée avec les microsphères fluorescentes en présence de vapeurs statiques de différents solvants volatils, à savoir d'éthanol, de toluène, d'acétone et de méthyléthylacétone. On constate ainsi qu'au bout d'une heure, la fluorescence du polymère est presque complètement éteinte en présence des vapeurs statiques de nitrobenzène, alors que la fluorescence ne varie que de ± 10% en présence de vapeurs de solvants organiques usuels, dont les pressions de vapeurs saturantes sont pourtant beaucoup plus élevées. De plus, l'atténuation de fluorescence par les vapeurs de nitrobenzène est très rapide. Le signal chute instantanément lorsque le dépôt est introduit dans la cuve et 50% de la fluorescence initiale sont éteints en 5 minutes. Les microsphères fluorescentes décrites sont ainsi capables de signaler la présence de vapeurs du composé nitroaromatique et de servir pour des applications en détection chimique.

### RÉFÉRENCES

[1] Monfort-Windels, F., Lumière et matière : des interactions au service de la lutte contre la contre-façon. L'Actualité Chimique 2007, 308-309, 108-110.
[2] Théry, E., La trace mise en lumière dans l'enquête policière. L'Actualité Chimique 2007, 308-309, 111-112.
[3] Obert, E.; LeBarny, P. Polymère fluorescent pour capteur physico-chimique. FR-A1-2900408, 2006.
[4] Jenkins, A. L.; Uly, O. M.; Murray, G. M., Polymer-based lanthanide luminescent sensor for detection of the hydrolysis product of the nerve agent Soman in water. Anal. Chem. 1999, 71, 373-378.
[5] Jenkins, A. L.; Yin, R.; Jensen, J. L., Molecularly imprinted polymer sensors for pesticide and insecticide detection in water. Analyst 2001, 126, 798-802.
[6] Hairault, L.; Pasquinet, E.; Montméat, P.; Moreau, J.; Lère-Porte, J.-P.; Wakim, S.; Serein-Spirau, F. Chemical sensors comprising fluorescent conjugated polymers as sensitive materials, and the use of thereof for the detection or dosage of nitrated compounds. Demande WO-A-2005103653, 2005.
[7] Campbell, A. I.; Bartlett, P., Fluorescent Hard-Sphere Polymer Colloids for Confocal Microscopy. Journal of Colloid and Interface Science 2002, 256, 325-330.
[8] Charreyre, M.-T.; Mandrand, B.; Martinho, J. M. G.; Relogio, P.; Sequeira, F. J. P. Polymères fluorescents en solution aqueuse et procédé de préparation de polymères fluorescents solubles en solution aqueuse. FR-A1-2887892, 2005.
[9] Waters, J., F. Aqueous dispersion of fluorescent pigments. Demande WO-A-1996/031565, 1996.
[10] Uchiyama, S.; Matsumura, Y.; Silva, A. P. d.; Iwai, K., Fluorescent molecular thermometers based on polymers showing temperature-induced phase transitions and labeled with polarity-responsive benzofurazans. Analytical Chemistry 2003, 75, 5926-5935.
[11] Trenor, S. R.; Schultz, A. R.; Love, B. J.; Long, T. E., Coumarins in polymers : From light harvesting to photo-cross-linkable tissue scaffolds. Chemical Reviews 2004, 104, (3059-3077).
[12] Bardez, E.; Boutin, P.; Valeur, B., Photoinduced biprotonic transfer in 4-methylumbelliferone. Chemical Physics Letters 1992, 191, 142-148.
[13] Cohen, B.; Ruppert, D., Excited state proton-transfer reactions of coumarin 4 in protic solvents. Journal of Physical Chemistry A 2001, 105, 7157-7164.
[14] Schulman, S. G.; Rosenberg, L. S., Tautomerization kinetics of 7-hydroxy-4-methylcoumarin in the lowest excited singlet state. Journal of Physical Chemistry 1979, 83, 447-451.
[15] Abdel-Mottaleb, M. S. A.; El-Sayed, B. A.; Abo-Aly, M. M.; El-Kady, M. Y., Fluorescence properties and excited state interactions of 7-hydroxy-4-methylcoumarin laser dye. Journal of Photochemistry and Photobiology, A : Chemistry 1989, 46, 379-390.
[16] Kudo, D.; Kon, A.; Yoshihara, S.; Kakizaki, I.; Sasaki, M.; Endo, M.; Takagaki, K., Effect of a hyaluronan synthase suppressor, 4-methylumbelliferone, on B16F-10 melanoma cell adhesion and locomotion. Biochemical and Biophysical Research Communications 2004, 321, 783-787.
[17] Nakamura, R.; Kuwabara, H.; Yoneda, M.; Yoshihara, S.; Ishikawa, T.; Miura, T.; Nozaka, H.; Nanashima, N.; Sato, T.; Nakamura, T., Suppression of matrix metalloproteinase-9 by 4-methylumbelliferone. Cell Biology International 2007, 31, 1022-1026.
[18] Yoshihara, S.; Kon, A.; Kudo, D.; Nakazawa, H.; Kakizaki, I.; Sasaki, M.; Endo, M.; Takagaki, K., A hyaluronan synthase suppressor, 4-methylumbelliferone, inhibits liver metastasis of melanoma cells. FEBS Letters 2005, 579, 2722-2726.
[19] Donglei Yu, M. S. L. X. S. L. M.-N. K.-H. L., Recent progress in the development of coumarin derivatives as potent anti-HIV agents. Medicinal Research Reviews 2003, 23, (3), 322-345.
[20] Pal, R.; Jr., W. A. P.; Ben-Yashar, V.; Wagner, R. R.; Barenholz, Y., Characterization of the fluorophore 4-heptadecyl-7-hydroxycoumarin: a -S-; -N- probe for the head-group region of lipid bilayers and biological membranes. Biochemistry 1985, 24 (3), 573-581.
[21] MolecularProbes. FluoSpheres and TransFluoSpheres Microspheres for Tracing.
[22] Chujo, Y.; Sada, K.; Saegusa, T., Polyoxazoline having a coumarin moiety as a pendant group. Synthesis and photogelation. Macromolecules 1990, 23, (10), 2693-2697.
[23] Rhum, D.; Matthews, R. S. Fluorescent polymers. US-A-5019350, 1991.
[24] Bouma, S. R.; Celebuski, J. E. 7-Hydroxy coumarins having substitutions in the 4 position. US-A-5208350, 1993.
[25] Pitschke, M.; Fels, A.; Schmidt, B.; Heiliger, L.; Kuckert, E.; Riesner, D., Polymeric fluorescent dyes for labeling of proteins and nucleic acids. Colloid and Polymer Science 1995, 273, 740-752.
[26] Rathbone, D. L.; Su, D.; Wang, Y.; Billington, D. C., Molecular recognition by fluorescent imprinted polymers. Tetrahedron Letters 2000, 41, 123-126.

## Revendications

1. Composé fluorescent polymérisable de la 7-hydroxycoumarine qui est l'umbelliférone-4-acétate d'éthyl-2-méthacrylate.

2. Procédé de préparation du composé selon la revendication 1, dans lequel on fait réagir l'acide umbelliférone-4-acétique avec un composé, qui est le méthacrylate de 2-hydroxyéthyle, comprenant d'une part une fonction hydroxy susceptible de réagir avec le groupement acide carboxylique de l'acide umbelliférone-4-acétique et d'autre part un groupement chimique polymérisable R qui est un groupement méthacrylate, moyennant quoi se produit un couplage du groupement acide carboxylique de l'acide umbelliférone-4-acétique avec ladite fonction hydroxy pour former l'umbelliférone-4-acétate d'éthyl-2-méthacrylate.

3. Polymère susceptible d'être obtenu par polymérisation d'un composé monomère fluorescent polymérisable de la 7-hydroxycoumarine répondant à la formule (I) suivante : 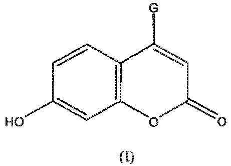 dans laquelle G est un substituant comprenant un groupement chimique polymérisable R choisi parmi les groupements vinyliques, styréniques, diéniques, acryliques, et méthacryliques ;
et éventuellement d'au moins un autre monomère polymérisable (IV) .

4. Polymère selon la revendication 3, dans lequel le composé de formule (I) est l'umbelliférone-4-acétate d'éthyl-2-méthacrylate.

5. Polymère selon l'une quelconque des revendications 3 à 4, qui est un homopolymère, susceptible d'être obtenu par polymérisation d'un composé monomère fluorescent de formule (I).

6. Polymère selon la revendication 5, qui est un homopolymère susceptible d'être obtenu par polymérisation de l'umbelliférone-4-acétate d'éthyl-2-méthacrylate.

7. Polymère selon l'une quelconque des revendications 3 à 4 qui est un copolymère statistique, alterné ou séquencé susceptible d'être obtenu par polymérisation d'au moins un composé monomère fluorescent de formule (I) et d'au moins un autre monomère copolymérisable.

8. Polymère selon la revendication 7, dans lequel l'autre monomère copolymérisable est choisi parmi les monomères acryliques, les monomères styréniques, et les monomères vinyliques.

9. Polymère selon la revendication 8, qui est un copolymère, de préférence un copolymère statistique, susceptible d'être obtenu par copolymérisation de l'umbelliférone-4-acétate d'éthyl-2-méthacrylate, du méthacrylate de 2-hydroxyéthyle, et du divinylbenzène, ou un copolymère, de préférence un copolymère statistique, susceptible d'être obtenu par copolymérisation de l' umbelliférone-4-acétate d'éthyl-2-méthacrylate, de la 4-vinylpyridine, et du divinylbenzène.

10. Polymère selon l'une quelconque des revendications 3 à 9, qui se présente sous la forme de sphères, notamment de microsphères d'un diamètre de 0,1 à 500 µm, de préférence de 0,1 à 200 µm.

11. Polymère selon l'une quelconque des revendications 3 à 10, qui se présente sous la forme d'un film mince déposé sur au moins une surface d'un substrat, ou d'un film épais.

12. Capteur comprenant le polymère selon l'une quelconque des revendications 3 à 11 en tant que matériau sensible.

13. Capteur selon la revendication 12, qui est un capteur optique dont le fonctionnement est basé sur la mesure des variations de l'intensité de la fluorescence émise par le polymère.

14. Utilisation du capteur selon la revendication 12 ou 13 pour la détection et/ou le dosage d'un ou plusieurs composés, molécules, cibles.

15. Utilisation selon la revendication 14 dans laquelle le ou lesdits composé(s) cible(s) est(sont) en phase vapeur, sous forme gazeuse.

16. Utilisation selon l'une quelconque des revendications 14 à 15 dans laquelle le(s) composé(s) cible(s) est(sont) choisi(s) parmi les composés nitrés et les composés organophosphorés.

17. Utilisation selon l'une quelconque des revendications 14 à 16 pour la détection et/ou le dosage d'explosifs ou de composés toxiques en particulier neurotoxiques.

18. Utilisation du polymère selon l'une quelconque des revendications 3 à 11 pour la réalisation de sondes fluorescentes, pour la réalisation de marqueurs biologiques, pour la réalisation de matériaux fluorescents, photoluminescents et/ou radioluminescents et/ou cathodoluminescents.

## Patentansprüche

1. Polymerisierbare fluoreszierende Verbindung des 7-Hydroxycumarins, die das Umbelliferon-4-acetat-Ethyl-2-methacrylat ist.

2. Herstellungsverfahren der Verbindung nach Anspruch 1, bei dem man die Umbelliferon-4-ethansäure mit einer Verbindung reagieren lässt, welche das 2-Hydroxyethyl-methacrylat ist, einerseits eine Hydroxyfunktion umfassend, geeignet mit der Carboxylsäuregruppe der Umbelliferon-4-ethansäure zu reagieren, und andrerseits eine chemisch polymerisierbare Gruppe R, die eine Methacrylat-Gruppe ist, wodurch sich eine Kopplung der Carboxylsäuregruppe der Umbelliferon-4-ethansäure mit der genannten Hydroxyfunktion ereignet, um das Umbelliferon-4-acetat-Ethyl-2-methacrylat zu bilden.

3. Polymer, herstellbar durch Polymerisation einer fluoreszierenden monomeren polymerisierbaren Verbindung des 7-Hydroxycumarin, folgender Formel (I) entsprechend: 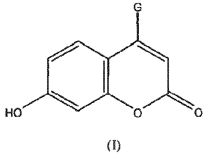 in der G ein Substituent ist, der eine chemisch polymerisierbare Gruppe R enthält, ausgewählt unter den Vinyl-, Styrol-, Dien-, Acryl- und Methacryl-Gruppen; und eventuell wenigstens ein anderes polymerisierbares Monomer (IV).

4. Polymer nach Anspruch 3, bei dem die Verbindung der Formel (I) das Umbelliferon-4-acetat-Ethyl-2-methacrylat ist.

5. Polymer nach einem der Ansprüche 3 bis 4, das ein Homopolymer ist, herstellbar durch Polymerisierung einer fluoreszierenden monomeren Verbindung der Formel (I).

6. Polymer nach Anspruch 5, das ein Homopolymer ist, herstellbar durch Polymerisierung von Umbelliferon-4-acetat-Ethyl-2-methacrylat.

7. Polymer nach einem der Ansprüche 3 bis 4, das ein statistisches, alternierendes oder sequenziertes Copolymer ist, herstellbar durch Polymerisation von wenigstens einer fluoreszierenden monomeren Verbindung der Formel (I) und wenigstens einem anderen copolymerisierbaren Monomer.

8. Polymer nach Anspruch 7, bei dem das andere copolymerisierbare Monomer ausgewählt wird unter den Acryl-, den Styrol- und den Vinylmonomeren.

9. Polymer nach Anspruch 8, das ein Copolymer, vorzugsweise ein statistisches Copolymer, ist herstellbar durch Copolymerisation des Umbelliferon-4-acetat-Ethyl-2-methacrylats, des 2-Hydroxyethylmethacrylats, und des Divinylbenzols, oder ein Copolymer, vorzugsweise ein statistisches Copolymer, ist herstellbar durch Copolymerisation des Umbelliferon-4-acetat-Ethyl-2-methacrylats, des 4-Vinylpyridins, und des Divinylbenzols.

10. Polymer nach einem der Ansprüche 3 bis 9, das sich in Form von Kugeln, insbesondere Mikrokugeln mit einem Durchmesser von 0,1 bis 500 µm, vorzugsweise 0,1 bis 200 µm, präsentiert.

11. Polymer nach einem der Ansprüche 3 bis 10, das sich in Form eines dünnen Films, abgeschieden auf wenigstens einer Fläche eines Substrats, oder eines dicken Films präsentiert.

12. Sensor der den Polymer nach einem der Ansprüche 3 bis 11 als empfindlichem Material umfasst.

13. Sensor nach Anspruch 12, der ein optischer Sensor ist, dessen Funktionsweise auf dem Messen der Veränderungen der durch das Polymer abgestrahlten Fluoreszenz beruht.

14. Verwendung des Sensors nach Anspruch 12 oder 13 zur Detektion und/oder Dosierung von einer oder mehreren Target-Verbindungen-Molekülen.

15. Verwendung nach Anspruch 14, bei der die genannte(n) Target-Verbindung(en) in der Dampfphase in Gas Form, ist (sind).

16. Verwendung nach einem der Ansprüche 14 bis 15, bei der die Target-Verbindung(en) ausgewählt wird (werden) unter den Nitroverbindungen und den organischen Phosphorverbindungen.

17. Verwendung nach einem der Ansprüche 14 bis 16 für die Detektion und/oder die Dosierung von Sprengstoffen oder von toxischen, insbesondere neurotoxischen Verbindungen.

18. Verwendung des Polymers nach einem der Ansprüche 3 bis 11 für die Herstellung von Fluoreszenzsonden, für die Herstellung von Biomarkern, für die Herstellung von fluoreszierenden, photolumineszenten und/oder radiolumineszenten und/oder kathodolumineszenten Materialien.

## Claims

1. A polymerizable fluorescent compound of 7-hydroxycoumarin which is Ethyl-2-methacrylate Umbelliferone-4-acetate.

2. A method for preparing the compound according to claim 1, wherein umbelliferone-4-acetic acid is reacted with a compound, which is 2-hydroxyethyl methacrylate, comprising a hydroxyl function capable of reacting with the carboxylic acid group of umbelliferone-4-acetic acid on the one hand, and a polymerizable chemical group R which is a methacrylate group on the other hand, whereby coupling of the carboxylic acid group of umbelliferone-4-acetic acid with said hydroxyl function occurs to form Ethyl-2-methacrylate Umbelliferone-4-acetate.

3. A polymer which is obtainable by polymerization of polymerizable fluorescent monomer compound of 7-hydroxycoumarin having the following formula (I): 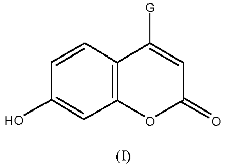 wherein G is a substituent comprising a polymerizable chemical group R selected from vinylic, styrenic, dienic, acrylic and methacrylic groups;
and optionally of at least one other polymerizable monomer (IV).

4. The polymer according to claim 3, wherein the compound of formula (I) is Ethyl-2-methacrylate Umbelliferone-4-acetate.

5. The polymer according to any one of claims 3 to 4, which is a homopolymer, obtainable by polymerization of a fluorescent monomer compound of formula (I).

6. The polymer according to claim 5, which is a homopolymer obtainable by polymerization of Ethyl-2-methacrylate Umbelliferone-4-acetate.

7. The polymer according to any one of claims 3 to 4, which is a random, alternating or block copolymer obtainable by polymerization of at least one fluorescent monomer compound of formula (I) and of at least one other copolymerizable monomer.

8. The polymer according to claim 7, wherein the other copolymerizable monomer is selected from acrylic monomers, styrenic monomers and vinylic monomers.

9. The polymer according to claim 8, which is a copolymer, preferably a random copolymer, obtainable by copolymerization of Ethyl-2-methacrylate Umbelliferone-4-acetate, 2-hydroxyethyl methacrylate, and divinylbenzene, or a copolymer, preferably a random copolymer, obtainable by copolymerization of Ethyl-2-methacrylate Umbelliferone-4-acetate, 4-vinyl pyridine, and divinylbenzene.

10. The polymer according to any one of claims 3 to 9, which appears in the form of spheres, notably of microspheres with a diameter from 0.1 to 500 µm, preferably from 0.1 to 200 µm.

11. The polymer according to any one of claims 3 to 10, which appears in the form of a thin film deposited on at least one surface of a substrate, or of a thick film.

12. A sensor comprising the polymer according to any one of claims 3 to 11 as a sensitive material.

13. The sensor according to claim 12, which is an optical sensor, the operation of which is based on the measurement of the variations of the intensity of the fluorescence emitted by the polymer.

14. The use of the sensor according to claim 12 or 13 for detecting and/or assaying one or several target compound(s), molecule(s).

15. The use according to claim 14 wherein said target compound(s) is(are) in the vapor phase, in a gaseous form.

16. The use according to any one of claims 14 to 15 wherein the target compound(s) is(are) selected from nitrated compounds and organophosphorus compounds.

17. The use according to any one of claims 14 to 16 for detecting and/or assaying explosives or toxic, in particular neurotoxic, compounds.

18. The use of the polymer according to any one of claims 3 to 11 for making fluorescent probes, for making biological markers, for making fluorescent, photoluminescent, and/or radioluminescent and/or cathodoluminescent materials.
